# FASCICULE DE BREVET EUROPEEN

(11) **EP 1 457 195 B1**
(45) Date de publication et mention de la délivrance du brevet: **13.06.2007**
(21) Numéro de dépôt: 04290371.6
(22) Date de dépôt: 12.02.2004
(51) Int. Cl.: A61K 8/73, A61K 8/81, A61Q 5/00, A61Q 5/04, A61Q 5/06, A61Q 5/08, C11D 3/22, C11D 3/37

(54) **Compositions cosmétiques contenant un copolymère d'acide méthacrylique, une cyclodextrine et un tensioactif et leurs utilisations**
Kosmetische Zusammensetzungen enthaltend ein Copolymer aus Methacrylsäure, Cyclodextrin und ein Tensid sowie deren Verwendungen
Cosmetic compositions containing a copolymer of methacrylic acid, cyclodextrine and a surfactant as well as their uses

(30) Priorité: 11.03.2003 FR 0302995
(43) Date de publication de la demande: 15.09.2004
(73) Titulaire: L'ORÉAL, 75008 Paris (FR)
(72) Inventeur: Müller, Rainer, 76344 Leopoldshafen (DE); Maubru, Mireille, 78400 Chatou (FR)
(74) Mandataire: Le Blainvaux Bellegarde, Françoise

(56) Documents cités:
- WO-A-01/76552
- WO-A-98/03155
- WO-A-99/55951
- DE-A- 10 043 118
- FR-A- 2 519 863
- GB-A- 2 344 827
- US-A1- 2002 086 809

## Description

La présente invention a pour objet des compositions comprenant dans un milieu aqueux physiologiquement acceptable au moins une cyclodextrine, au moins un tensioactif et au moins un copolymère réticulé acide méthacrylique / acrylate d'alkyle en C₁-C₄. Elle a aussi pour objet des compositions comprenant en outre au moins un agent de conditionnement. L'invention a également pour objet l'utilisation du copolymère réticulé acide méthacrylique / acrylate d'alkyle en C₁-C₄ en tant qu'agent de suspension du complexe formé de la cyclodextrine et du tensioactif.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents de conditionnement, notamment insolubles, pour faciliter le démêlage des cheveux et pour leur communiquer douceur, brillance et souplesse.

Compte tenu du caractère insoluble de certains agents de conditionnement tels que par exemple les silicones ou les huiles, on cherche à maintenir les agents de conditionnement en dispersion régulière dans le milieu sans cependant faire chuter la viscosité et les propriétés détergentes et moussantes des compositions. Les agents de conditionnement notamment les silicones ou les huiles doivent également être véhiculés sur les matières kératiniques traitées en vue de leur conférer, suivant l'application, des propriétés de douceur, de brillance et de démêlage.

On sait également que les produits en particulier cosmétiques ayant un aspect ou un effet irisé, moiré ou métallisé, sont largement appréciés par les consommateurs pour leur aspect esthétique et donnant une apparence de richesse au produit. Les agents qui apportent cet effet sont des agents de nacrage ou agent nacrants comprenant généralement des cristaux qui restent dispersés dans les compositions et qui réfléchissent la lumière.
Les dérivés d'esters à longue chaîne sont largement utilisés pour nacrer les compositions notamment cosmétiques. Cependant, ces dérivés peuvent présenter des problèmes de cristallisation qui entraînent une évolution de la viscosité des compositions au cours du temps.

On connaît également les dérivés d'éthers ou de thioéthers à longue chaîne tels que ceux décrits dans les demandes EP457688 et WO98/03155. Cependant, ces derniers agents opacifient les compositions sans apporter de nacrage aux compositions ou pas suffisamment.

Il a été constaté que ces agents nacrants, du fait de leur faible densité, présentaient souvent l'inconvénient de remonter à la surface du shampooing et d'y former une couche inesthétique pour le consommateur.

De plus ces composés à chaînes grasses présentent dans certains cas l'inconvénient d'apporter un toucher chargé aux cheveux, un manque de légèreté, de volume de la chevelure .

Il existe donc toujours un besoin pour de nouveaux agents nacrants qui ne présentent pas les inconvénient cités ci-dessus et qui permettent également l'utilisation d'agents de conditionnement insolubles et en particulier les silicones.

Pour épaissir et stabiliser les compositions cosmétiques contenant des agents conditionneurs insolubles, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

On connaît dans l'état de la technique des compositions cosmétiques en particulier détergentes contenant un copolymère réticulé d'acide méthacrylique et d'acrylate d'alkyle comme agent de stabilisation ou de suspension d'ingrédients insolubles dans l'eau comme des silicones ou des corps gras. De telles compositions ont été décrites notamment dans la demande de brevet WO01/76552. Les qualités de mousse et les propriétés cosmétiques obtenues avec ces compositions ne sont pas encore suffisamment satisfaisantes.

La demande WO 99/55951 A1 divulgue des compositions pour défroisser et désodoriser les textiles comprenant une cyclodextrine non complexée et un polymère constitué de monomères hydrophobes et de monomères hydrophiles. Parmi la liste de copolymères, on trouve un copolymère acide acrylique/acrylate de butyle, et un copolymère d'acrylate d'éthyle, d'acide methacrylique, de méthacrylate de méthyle et d'acide acrylique. Ce document ne décrit pas de copolymère réticulé acide methacrylique / acrylate d'alkyle en C1-C4.

La demande DE 100 43 118 A1 décrit des compositions de hautes viscosité comprenant au moins une substance déodorantes et ayant une viscosité particulière. La substance déodorante peut être associée a une cyclodextrine. La composition est épaissie avec un épaississant organique ou inorganique. Les polymères organiques peuvent être d'origine naturelle ou synthétique, réticulés ou non réticulés. Ce document ne décrit pas de copolymère réticulé acide methacrylique / acrylate d'alkyle en C1-C4.

La demande GB 2 344 827 décrit des compositions détergentes comprenant un polymère hydrophile, un cyclohexane volatile et éventuellement un tensioactif et une cyclodextrine. Ce document décrit des polymères d'acide acrylique mais pas d'acide méthylique, ni de copolyméres. Ce document ne décrit pas de copolymère réticulé acide methacrylique / acrylate d'alkyle en C1-C4.

La demanderesse a découvert qu'il était possible de formuler des compositions notamment cosmétique pour le traitement des matières kératiniques, en particulier des shampooings présentant un aspect nacré tout en ayant les propriétés esthétiques et cosmétiques recherchées, en utilisant dans ces compositions au moins une cyclodextrine ou l'un de ses dérivés, au moins un tensioactif et au moins un polymère particulier.

L'invention a pour objet des compositions notamment cosmétiques comprenant dans un milieu aqueux notamment physiologiquement acceptable et en particulier cosmétiquement acceptable au moins un tensioactif, au moins une cyclodextrine ou l'un de ses dérivés et au moins un copolymère réticulé acide méthacrylique /acrylate d'alkyle en C₁-C₄.

La présence de ce copolymère ne diminue pas les propriétés cosmétiques des compositions et peut même les améliorer, notamment en ce qui concerne le lissage, le volume et/ou la discipline.

Les compositions présentent une très bonne homogénéité et une bonne stabilité du nacrage, ainsi qu'une viscosité satisfaisante pour l'application sur les matières kératiniques. Il n'y a pas de déphasage (séparation de phase).

En particulier, il ne se produit aucun relargage ou épaississement incontrôlé de la composition au cours du temps. Les compositions présentent enfin une texture non filante et fondante. La mousse se rince facilement.

Un autre objet de l'invention est constitué par le procédé de lavage et/ou de conditionnement mettant en oeuvre de telles compositions.

Un autre objet de l'invention concerne l'utilisation d'un copolymère réticulé acide méthacrylique / acrylate d'alkyle en C₁-C₄ dans, ou pour la fabrication d'une composition cosmétique comprenant au moins une cyclodextrine et au moins un tensioactif.

D'autres objets de l'invention apparaîtront à la lecture de la description et des exemples qui suivent.

Selon la présente invention, par matières kératiniques, on comprend les cheveux, les cils, les sourcils, la peau, les ongles, les muqueuses ou le cuir chevelu et plus particulièrement les cheveux.
Par le terme "agent de nacrage" ou "agent nacrant", on entend un agent produisant un aspect ou un effet nacré, irisé, moiré ou métallisé.

L'une des caractéristiques essentielles de l'invention est la présence d'un copolymère réticulé d'acide méthacrylique et d'acrylate d'alkyle en C₁-C₄ .

L'acide méthacrylique est présent de préférence dans des quantités allant de 20 à 80% en poids et plus particulièrement de 25 à 70 % en poids et encore plus particulièrement de 35 à 60% en poids par rapport au poids total du copolymère.

L'acrylate d'alkyle est présent de préférence dans des quantités allant de 15 à 80% en poids et plus particulièrement de 25 à 75 % en poids et encore plus particulièrement de 40 à 65% en poids par rapport au poids total du copolymère. Il est choisi notamment parmi l'acrylate de méthyle, acrylate d'éthyle ou l'acrylate de butyle et plus particulièrement l'acrylate d'éthyle.

Ce copolymère est partiellement ou totalement réticulé par au moins un agent réticulant classique. Les agents réticulants sont notamment des composés polyinsaturés, en particulier éthylèniquement polyinsaturés. Ces composés sont notamment les polyalcényléthers de sucrose ou de polyols, les diallylphtalates, le divinylbenzène, le (méth)acrylate d'allyle, di(méth)acrylate d'éthylèneglycol, le méthylène bis-acrylamide, le tri(méth)acrylate de triméthylol propane, l'itaconate de diallyle, le fumarate de diallyle, le maléate de diallyle, le (méth)acrylate de zinc, les dérivés d'huile de ricin ou de polyols fabriqués à partir d'acides carboxyliques insaturés.
Comme agent réticulant, on peut également utiliser des composés monomères insaturés et comportant un groupe réactif susceptible de réagir avec une insaturation pour former un copolymère réticulé.
La teneur en agent réticulant varie en général de 0,01 à 5% en poids et de préférence de 0,03 à 3% en poids et encore plus particulièrement de 0,05 à 1 % en poids par rapport au poids total du copolymère.

Selon une forme particulièrement préférée, le copolymère de l'invention peut se présenter notamment sous forme de dispersion dans l'eau. La taille moyenne en nombre des particules de copolymère dans la dispersion est généralement comprise entre 10 et 500 nm et de préférence entre 20 et 200 nm et plus préférentiellement de 50 à 150 nm.

Ces copolymères sont notamment décrit dans la demande WO01/76552.

On utilisera plus particulièrement le copolymère acide méthacrylique/acrylate d'éthyle réticulé sous forme de dispersion aqueuse à 30% fabriqué et vendu sous le nom CARBOPOL AQUA SF-1 par la société NOVEON.

La concentration en copolymère réticulé est généralement comprise entre 0,01 et 10% en poids par rapport au poids total de la composition et de préférence entre 0,1 et 5% en poids.

Les cyclodextrines sont notamment des oligosaccharides de formule : 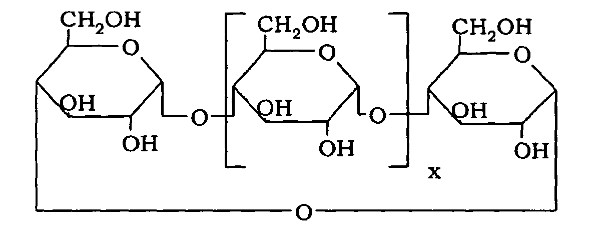 dans laquelle x peut être un nombre égal à 4 (ce qui correspond à l'α-cyclodextrine), à 5 (β-cyclodextrine) ou à 6 (γ-cyclodextrine).

On peut notamment utiliser une beta-cyclodextrine vendu par la société WACKER sous la dénomination CAVAMAX W7 et une gamma cyclodextrine vendu par la société WACKER sous la dénomination CAVAMAX W8.

Les dérivés de cyclodextrines sont par exemple les méthyl cyclodextrines tels que la méthyl-beta-cyclodextrine commercialisée par la société WACKER sous la dénomination CAVASOL W7).

Selon l'invention, la ou les cyclodextrines peuvent représenter de 0,2% à 30 % en poids, de préférence de 1 % à 15 % en poids, et encore plus préférentiellement de 1,5 % à 10 % en poids, du poids total de la composition finale.

Les compositions de l'invention comprennent en outre au moins un tensioactif qui est généralement présent en une quantité comprise entre 0,2% et 40% en poids environ, de préférence entre 1 % et 35% et encore plus préférentiellement entre 1,5 % et 30%, par rapport au poids total de la composition.

La cyclodextrine ou l'un de ses dérivés et le tensioactif sont présents de préférence à une concentration efficace pour nacrer la composition et/ou pour former un complexe insoluble dans la composition entre la cyclodextrine et le tensioactif ou les tensioactifs.

Le rapport tensioactif/cyclodextrine peut varier de 0,01 à 300, de préférence de 0,1 à 100 et plus particulièrement de 0,3 à 25.

Les tensioactifs convenant à la mise en oeuvre de la présente invention peuvent être de toute nature et de préférence sont solubles dans l'eau à température ambiante :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkylamido(C₆-C₂₄) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools polyéthoxylés, polypropoxylés ou polyglycérolés, les alpha-diols polyéthoxylés, polypropoxylés ou polyglycérolés, les alkylphénols polyéthoxylés, polypropoxylés ou polyglycérolés ou les acides gras polyéthoxylés, polypropoxylés ou polyglycérolés, tous ces composés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s) ou zwittérionique(s) :

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits commercialisés sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives :

R₂ -CONHCH₂CH₂ -N(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphodiacetate commercialisé sous la dénomination commerciale MIRANOL C2M concentré NP par la Société RHODIA CHIMIE.

### (iv) Tensioactifs cationiques :

Les tensioactifs cationiques peuvent être choisis parmi :
A) les sels d'ammonium quaternaires de la formule générale (XII) suivante : 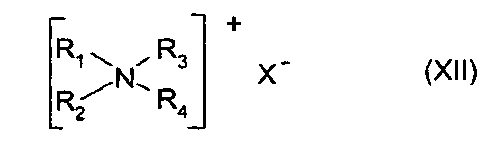 dans laquelle X⁻ est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de cétyl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (XIII) suivante : 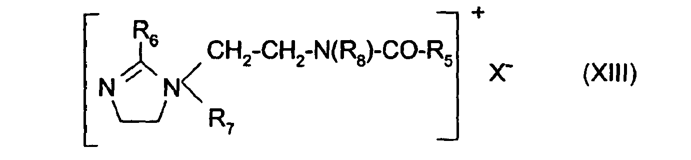 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (XIV) : 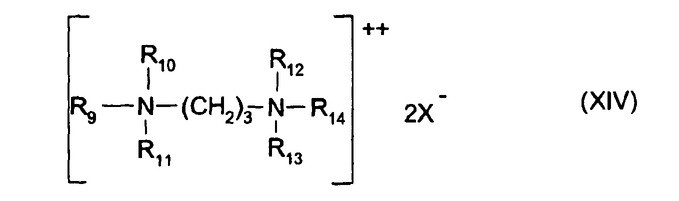 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (XV) suivante : 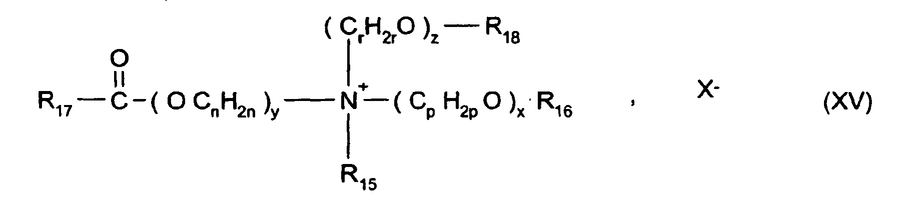 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 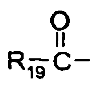
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 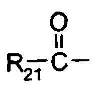
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 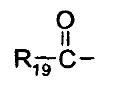
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 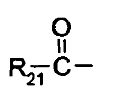
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société DEGUSSA-WITCO.

Parmi les sels d'ammonium quaternaire on préfère le chlorure de cétyltriméthylammonium, ou encore, le chlorure palmitamidopropyl triméthyl ammonium commercialisé sous la dénomination VARISOFT PA TC par la société DEGUSSA.

Les compositions comprennent de préférence au moins un tensioactif anionique.

On utilise de préférence comme agent tensioactif anionique les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélanges avec :
- soit un agent tensioactif amphotère tel que les dérivés d'amine dénommés disodiumcocoamphodiacetate ou sodiumcocoamphopropionate commercialisés notamment par la société RHODIA CHIMIE sous la dénomination commerciale "MIRANOL^{®} C2M CONCNP" en solution aqueuse à 38 % de matière active ou sous la dénomination MIRANOL^{®} C32;
- soit un agent tensioactif amphotère tel que les alkylbétaïnes en particulier la cocobétaïne commercialisée sous la dénomination "DEHYTON^{®} AB 30" en solution aqueuse à 32 % de MA par la société COGNIS ou tel que les alkyl (C₈-C₂₀) amidoalkyl (C₁-C₆) bétaïnes en particulier la TEGOBETAINE^{®} F 50 commercialisée par la société DEGUSSA.

Selon une variante préférée de l'invention, les compositions cosmétiques peuvent également contenir des agents de conditionnement des matières kératiniques.

Ces compositions, lorsqu'elles sont appliquées sur les cheveux, possèdent de bonnes propriétés de conditionnement des cheveux, c'est-à-dire que les cheveux traités sont lisses, se démêlent facilement, sont doux au toucher. Les cheveux ont un aspect naturel et non chargé.

Lorsque la composition contient au moins un agent de conditionnement, ils sont généralement choisis parmi les huiles de synthèse telles que les poly-α-oléfines, les huiles fluorées, les cires fluorées, les gommes fluorées, les esters d'acides carboxyliques, les polymères cationiques, les silicones, les huiles minérales, végétales ou animales, les céramides, les pseudocéramides et leurs mélanges.

Les polyoléfines sont de préférence des poly-α-oléfines et en particulier :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence allant de 1000 à 15000.
   A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.
   De tels produits sont vendus par exemple sous les dénominations ETHYLFLO par la société ETHYL CORP., et d'ARLAMOL PAO par la société ICI.

Les huiles minérales pouvant être utilisées dans les compositions de l'invention sont choisies préférentiellement dans le groupe formé par :
- les hydrocarbures, tels que l'hexadécane et l'huile de paraffine ;

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société NALCO, les polysaccharides cationiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement réticulés de sel de (méth)acryloyloxyéthyltriméthylammonium, vendus par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium). On peut également utiliser les polymères qui sont constitués de motifs récurrents répondant à la formule : 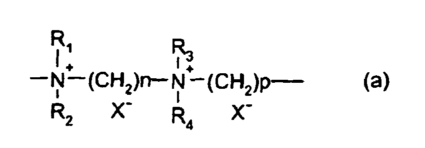 dans laquelle R₁, R₂, R₃ et R_{4,} identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Les silicones utilisables conformément à l'invention sont en particulier des polyorganosiloxanes insolubles dans la composition et peuvent se présenter sous forme d'huiles, de cires, de résines ou de gommes.

Les silicones insolubles dans l'eau sont insolubles dans l'eau à une concentration supérieure ou égale à 0,1% en poids dans l'eau à 25°C, c'est à dire qu'elles ne forment pas une solution isotrope transparente.

La viscosité des silicones est par exemple mesurée à 25°C selon la norme ASTM 445 Appendice C.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique : 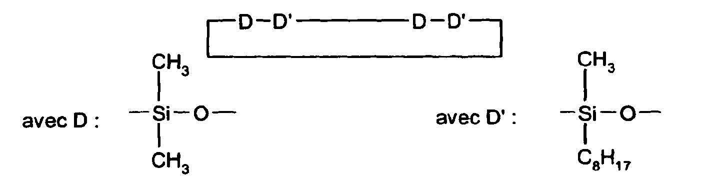
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s.

Parmi ces polyalkylsiloxanes, on peut citer à titre non limitatif les produits commerciaux suivants :
- les huiles de la série MIRASIL commercialisées par la société RHODIA CHIMIE, telles que par exemple l'huile MIRASIL DM 500 000 ;
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL WAX 9800 et 9801" par la société DEGUSSA qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles MIRASIL DPDM de RHODIA CHIMIE ;
. les huiles des séries RHODORSIL 70 633 et 763 de RHODIA CHIMIE ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société DEGUSSA ;
- des groupements hydroxylés comme les polyorganosiloxanes à fonction hydroxyalkyle décrits dans la demande de brevet français FR-A-85 16334 .
   - des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société DEGUSSA sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 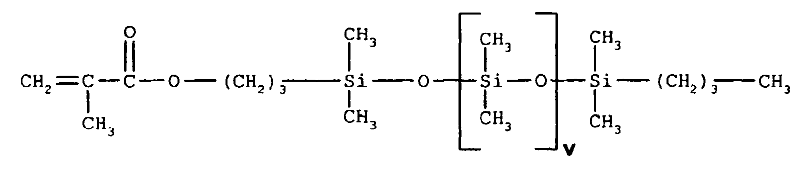
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise allant de 0,2 à 2,5 m²/s à 25° C telles que les huiles de la séries DC200 de DOW CORNING en particulier celle de viscosité 60 000 cSt, des séries MIRASIL DM et plus particulièrement l'huile MIRASIL DM 500 000 commercialisées par la société RHODIA CHIMIE ou l'huile de silicone AK 300.000 de la société WACKER, les polyalkylsiloxanes à groupements terminaux diméthylsilanol tels que les diméthiconol ou les polyalkylarylsiloxanes tels que l'huile MIRASIL DPDM commercialisée par la société RHODIA CHIMIE ;
- les polysiloxanes à groupements aminés tels que les amodiméthicones ou les triméthylsilylamodiméthicones ;

Selon la présente invention, les composés de type céramide sont notamment les céramides et/ou les glycocéramides et/ou les pseudocéramides et/ou les néocéramides, naturels ou synthétiques.

Des composés de type céramide sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131 dont les enseignements sont ici inclus à titre de référence.

Des composés de type céramides particulièrement préférés selon l'invention sont par exemple :
- le 2-N-linoléoylamino-octadécane-1,3-diol,
- le 2-N-oléoylamino-octadécane-1,3-diol,
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1 ,3-diol
- le (bis-(N-hydroxyéthyl N-cétyl) malonamide),
- le N-(2-hydroxyéthyl)-N-(3-cétyloxy-2-hydroxypropyl)amide d'acide cétylique
- le N-docosanoyl N-méthyl-D-glucamine
ou les mélanges de ces composés.

Selon l'invention, les agents conditionneurs peuvent représenter de 0,001 % à 10 % en poids, de préférence de 0,005 % à 5 % en poids, et encore plus préférentiellement de 0,01 % à 3 % en poids, du poids total de la composition finale.

Le milieu physiologiquement acceptable est de préférence constitué par de l'eau ou un mélange d'eau et de solvants cosmétiquement ou dermatologiquement acceptables tels que des monoalcools, des polyalcools, des éthers de glycol qui peuvent être utilisés seuls ou en mélange. L'eau représente de préférence de 30 à 98% en poids et de préférence de 50 à 98% en poids par rapport au poids total de la composition.

On peut citer plus particulièrement les monoalcools tels que l'éthanol, l'isopropanol, les polyalcools tels que le diéthylèneglycol, la glycérine, les éthers de glycol, les alkyléthers de glycol ou de diéthylèneglycol.

La composition de l'invention peut également contenir au moins un additif choisi parmi les séquestrants, les adoucissants, les modificateurs de mousse, les colorants, d'autres agents nacrants, les agents hydratants, les agents antipelliculaires ou anti-séborrhéiques, d'autres agents de mise en suspension, les acides gras à chaînes linéaires ou ramifiées en C₁₆-C₄₀, les hydroxyacides, les électrolytes, les épaississants, les esters d'acides gras, les parfums, les conservateurs, les filtres solaires, les protéines, les vitamines et provitamines, les polymères, et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion épaissie ou non ou de mousse.

Les compositions conformes à l'invention peuvent être utilisées pour le traitement des matières kératiniques telles que les cheveux, la peau, les cils, les sourcils, les ongles, les lèvres, le cuir chevelu et plus particulièrement les cheveux.

Les compositions peuvent également être utilisées pour le lavage et le nettoyage des matières kératiniques telles que les cheveux et la peau.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

En particulier, les compositions selon l'invention sont des compositions détergentes moussantes telles que des shampooings, des gels-douche et des bains moussants. Dans ce mode de réalisation de l'invention, les compositions comprennent au moins un tensioactif détergent.

Le ou les tensioactifs détergents peuvent être indifféremment choisis, seuls ou en mélanges, au sein des tensioactifs anioniques, amphotères, non ioniques, zwittérioniques et cationiques décrits ci-dessus.

La quantité minimale en tensioactif est celle juste suffisante pour conférer à la composition finale un pouvoir moussant et/ou détergent satisfaisant.

Ainsi, selon l'invention, le tensioactif détergent peut représenter de 3 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1 m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement un tensioactif cationique, sa concentration allant généralement de 0,1 à 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux. Les compositions sont de préférence liquides.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

L'invention a encore pour objet un procédé de traitement cosmétique des matières kératiniques telles que les cheveux consistant à appliquer sur ceux-ci une composition telle que définie précédemment puis à effectuer éventuellement un rinçage à l'eau, après un éventuel temps de pause.

L'invention va être maintenant plus complètement illustrée à l'aide des exemples suivants qui ne sauraient être considérés comme la limitant aux modes de réalisation décrits. Dans ce qui suit, MA signifie Matière Active.

### EXEMPLES 1 et 2

On a préparé deux shampooings de compositions suivantes :

| Composition | Exemple 1 | Exemple 2 |
|---|---|---|
| Lauryléthersulfate de sodium à 2.2 moles | 11.8 g M.A. | 15.5 g M.A. |
| d'oxyde d'éthylène | | |
| Cocoylbétaïne | 1.5 g M.A. | - |
| Cocoyl amidopropyl bétaïne | - | 2.4 g M.A. |
| Copolymère acide méthacrylique/ | 10 g | 11 g |
| acrylate d'éthyle en émulsion aqueuse à | | |
| 30% de matière active, commercialisé | | |
| par la société NOVEON | | |
| Cyclodextrine, vendue sous la | 1.5 g | 1.5 g |
| dénomination Cavamax W7 par la | | |
| société WACKER | | |
| Polydiméthylsiloxane de viscosité 500 | 1.5 g | - |
| 000 cSt, vendue sous la dénomination | | |
| Mirasil DM 500 000 par la société | | |
| RHODIA | | |
| Polydiméthylsiloxane de viscosité 300 000 | - | 2.7 g |
| cSt, vendue sous la dénomination DC200 | | |
| fluid 300 000 par la société DOW | | |
| CORNING | | |
| Hydroxyéthylcellulose réticulée à | 0.4 g | - |
| l'épichlorhydrine, quatemisée par la | | |
| triméthylamine, vendue sous la | | |
| dénomination JR 400 par la société | | |
| AMERCHOL | | |
| Gomme de guar modifiée par du chlorure | - | 0.1 g |
| de 2,3-époxypropyl triméthyl ammonium | | |
| vendue sous la dénomination Jaguar C13 | | |
| S par la société RHODIA | | |
| Conservateurs, parfum | q.s. | q.s. |
| Agent de pH q.s.p. | pH 7 | pH 7 |
| Eau déminéralisée q.s.p. | 100 g | 100 g |

Ces formulations ont un bel effet nacré, sont stables et présentent de bonnes propriétés cosmétiques.

### EXEMPLES 3 et 4

On a préparé les compositions de shampooing suivantes :

### En g

| | Exemple 3 | Exemple 4 |
|---|---|---|
| Polyquatemium-10 | 0,8g | 0,8g |
| Carbopol Aqua SF 1 | 2g | 3g |
| Cyclodextrine (Cavamax de WACKER | 2g | 2g |
| Sodium Laureth Sulfate (70 % m.a.) | 16g | 16g |
| Cocamidopropylbétaine (30 % m.a.) | 8g | 8g |
| PEG-60 Hydrogenated Castor Oil | 0,2g | 0,2g |
| Parfum | 0,7g | 0,7g |
| Olea europaea | 0,01 g | 0,01 g |
| Citrus limonum | 0,01 g | 0,01 g |
| DMDM Hydantoin/Sodium | | |
| Méthylparaben | 0,45g | 0,45g |
| Agent de pH (Acide Tartrique /Acide | | |
| Citrique/ Ammoniaque) qs | pH 5,5 | pH 5,5 |
| Eau déminéralisée qsp | 100 g | 100 g |
| aspect | nacré | nacré |
| test de conservation | stable 2 | stable 2 mois |
| | mois à | à 45°C |
| | 45°C | |

### EXEMPLE 5

On a préparé la composition de shampooing suivante :

| | |
|---|---|
| Eau déminéralisée | qsp 100 g |
| Sodium Laureth Sulfate (70 % m.a.) | 22,2g |
| Carbopol Aqua SF 1 | 5g |
| Hydroxyde de Sodium | 0,19g |
| Coco Betaine (30% ma) | 9g |
| Cétéaryl Sulfate de Sodium (90 % m.a.) | 0,75g |
| Cyclodextrine, vendue sous la dénomination Cavamax W7 par la | |
| société WACKER | 2,5g |
| Propylène Glycol | 0,1g |
| Piroctone Olamine | 0,5g |
| Dimethicone ( Mirasil DM 500.000 de la société RHODIA ) | 2g |
| Vitamines | 0, 2g |
| Extrait de lémon et jus de pomme | 0,6g |
| DMDM Hydantoin/Paraben/Phénoxyethanol | 0,5g |
| Parfum | qs |
| Agent de pH qs | pH 6,3 |
| aspect | nacré |
| test de conservation | Stable 2 mois à 45°C |

## Revendications

1. Composition cosmétique **caractérisée en ce qu'**elle comprend dans un milieu aqueux au moins un agent tensioactif, au moins une cyclodextrine ou l'un de ses dérivés et au moins un copolymère réticulé acide méthacrylique / acrylate d'alkyle en C₁-C₄.

2. Composition selon la revendication 1, **caractérisée par le fait que** les cyclodextrines ou l'un de leurs dérivés sont choisies parmi l'α-cyclodextrine, la β-cyclodextrine ou la γ-cyclodextrine ou l'un de leurs dérivés.

3. Composition selon la revendication 2, **caractérisée par le fait que** les cyclodextrines sont choisies parmi la β-cyclodextrine ou la γ-cyclodextrine.

4. Composition selon l'une quelconque des revendications 1 à 3, **caractérisée en ce que** la cyclodextrine ou l'un de ses dérivés représente de 0,2% à 30 % en poids, de préférence de 1 % à 15 % en poids, par rapport au poids total de la composition finale.

5. Composition selon l'une quelconque des revendications 1 à 4, **caractérisée en ce que** dans ledit copolymère, l'acide méthacrylique est présent dans des quantités allant de 20 à 80% en poids et plus particulièrement de 25 à 70 % en poids et encore plus particulièrement de 35 à 60% en poids par rapport au poids total du copolymère.

6. Composition selon l'une quelconque des revendications 1 à 5, **caractérisée en ce que** dans ledit copolymère, l'acrylate d'alkyle est présent dans des quantités allant de 15 à 80% en poids et plus particulièrement de 25 à 75 % en poids et encore plus particulièrement de 40 à 65% en poids par rapport au poids total du copolymère.

7. Composition selon l'une quelconque des revendications 1 à 6, **caractérisée en ce que** dans ledit copolymère, l'acrylate d'alkyle est choisi parmi l'acrylate de méthyle, l'acrylate d'éthyle ou l'acrylate de butyle.

8. Composition selon la revendication 7, **caractérisé en ce que** l'acrylate d'alkyle est l'acrylate d'éthyle.

9. Composition selon l'une quelconque des revendications 1 à 8, où le copolymère d'acide méthacrylique et d'acrylate d'alkyle en C₁-C₄ est réticulé par au moins un agent réticulant éthylèniquement polyinsaturé.

10. Composition selon la revendication 9, **caractérisée en ce que** la teneur en agent réticulant varie de 0,01 à 5% en poids et de préférence de 0,03 à 3% en poids et encore plus particulièrement de 0,05 à 1 % en poids par rapport au poids total du copolymère.

11. Composition selon l'une quelconque des revendications précédentes, **caractérisée en ce que** le copolymère réticulé acide méthacrylique et d'acrylate d'alkyle en C₁-C₄ est présent à une concentration comprise entre 0,01 et 10% en poids par rapport au poids total de la composition et de préférence entre 0,1 et 5% en poids.

12. Composition selon l'une quelconque des revendications précédentes, **caractérisée en ce que** le tensioactif est un tensioactif anionique.

13. Composition selon l'une quelconque des revendications 1 à 12, **caractérisée en ce que** le tensioactif est présent allant de 0,2% à 40% en poids, de préférence de 1 % à 35% par rapport au poids total de la composition.

14. Composition selon l'une quelconque des revendications précédentes, **caractérisée en ce qu'**elle comprend en outre au moins un agent de conditionnement.

15. Composition selon la revendication précédente, **caractérisée en ce que** l'agent de conditionnement est choisi parmi les poly-α-oléfines, les huiles fluorées, les cires fluorées, les gommes fluorées, les esters d'acides carboxyliques, les silicones, les polymères cationiques, les huiles minérales, végétales ou animales, les céramides, les pseudocéramides, et leurs mélanges.

16. Composition selon la revendication précédente, **caractérisée en ce** les agents conditionneurs représentent de 0,001 % à 10 % en poids, de préférence de 0,005 % à 5 % en poids, et encore plus préférentiellement de 0,01 % à 3 % en poids, du poids total de la composition.

17. Composition selon l'une quelconque des revendications précédentes, **caractérisée par le fait qu'**elle se présente sous forme de gel, de lait, de crème, de lotion ou de mousse.

18. Composition selon l'une quelconque des revendications précédentes, **caractérisée par le fait qu'**elle constitue une composition détergente moussante telles que des shampooings, des gels-douche et des bains moussants.

19. Composition selon l'une quelconque des revendications précédentes, **caractérisée par le fait qu'**elle constitue une composition d'après-shampooing à rincer ou non, des compositions pour permanente, défrisage, coloration ou décoloration, des compositions à appliquer avant ou après une coloration, une décoloration, une permanente ou un défrisage ou encore entre les deux étapes d'une permanente ou d'un défrisage.

20. Procédé de traitement cosmétique des matières kératiniques en particulier des cheveux, **caractérisé par le fait qu'**il consiste à appliquer sur lesdites matières une composition telle que définie selon l'une quelconque des revendications 1 à 20 puis à effectuer éventuellement un rinçage à l'eau.

21. Utilisation d'un copolymère réticulé acide méthacrylique / acrylate d'alkyle en C₁-C₄ dans, ou pour la fabrication d'une composition cosmétique comprenant au moins une cyclodextrine et au moins un tensioactif.

## Claims

1. Cosmetic composition, **characterized in that** it comprises, in an aqueous medium, at least one surfactant, at least one cyclodextrin or a derivative thereof and at least one methacrylic acid/C₁-C₄ alkyl acrylate crosslinked copolymer.

2. Composition according to Claim 1, **characterized in that** the cyclodextrins or a derivative thereof are chosen from α-cyclodextrin, β-cyclodextrin and y-cyclodextrin, or a derivative thereof.

3. Composition according to Claim 2, **characterized in that** the cyclodextrins are chosen from β-cyclodextrin and γ-cyclodextrin.

4. Composition according to any one of Claims 1 to 3, **characterized in that** the cyclodextrin or a derivative thereof represents from 0.2% to 30% by weight and preferably from 1% to 15% by weight relative to the total weight of the final composition.

5. Composition according to any one of Claims 1 to 4, **characterized in that**, in the said copolymer, the methacrylic acid is present in amounts ranging from 20% to 80% by weight, more particularly from 25% to 70% by weight and even more particularly from 35% to 60% by weight relative to the total weight of the copolymer.

6. Composition according to any one of Claims 1 to 5, **characterized in that**, in the said copolymer, the alkyl acrylate is present in amounts ranging from 15% to 80% by weight, more particularly from 25% to 75% by weight and even more particularly from 40% to 65% by weight relative to the total weight of the copolymer.

7. Composition according to any one of Claims 1 to 6, **characterized in that**, in the said copolymer, the alkyl acrylate is chosen from methyl acrylate, ethyl acrylate and butyl acrylate.

8. Composition according to Claim 7, **characterized in that** the alkyl acrylate is ethyl acrylate.

9. Composition according to any one of Claims 1 to 8, in which the copolymer of methacrylic acid and of a C₁-C₄ alkyl acrylate is crosslinked with at least one polyethylenically unsaturated crosslinking agent.

10. Composition according to Claim 9, **characterized in that** the content of crosslinking agent ranges from 0.01% to 5% by weight, preferably from 0.03% to 3% by weight and even more particularly from 0.05% to 1% by weight relative to the total weight of the copolymer.

11. Composition according to any one of the preceding claims, **characterized in that** the crosslinked copolymer of methacrylic acid and of a C₁-C₄ alkyl acrylate is present in a concentration of between 0.01% and 10% by weight and preferably between 0.1% and 5% by weight relative to the total weight of the composition.

12. Composition according to any one of the preceding claims, **characterized in that** the surfactant is an anionic surfactant.

13. Composition according to any one of Claims 1 to 12, **characterized in that** the surfactant is present in an amount ranging from 0.2% to 40% by weight and preferably from 1% to 35% by weight relative to the total weight of the composition.

14. Composition according to any one of the preceding claims, **characterized in that** it also comprises at least one conditioner.

15. Composition according to the preceding claim, **characterized in that** the conditioner is chosen from poly-α-olefins, fluoro oils, fluoro waxes, fluoro gums, carboxylic acid esters, silicones, cationic polymers, mineral, plant or animal oils, ceramides and pseudoceramides, and mixtures thereof.

16. Composition according to the preceding claim, **characterized in that** the conditioners represent from 0.001% to 10% by weight, preferably from 0.005% to 5% by weight and even more preferably from 0.01% to 3% by weight relative to the total weight of the composition.

17. Composition according to any one of the preceding claims, **characterized in that** it is in the form of a gel, a milk, a cream, a lotion or a mousse.

18. Composition according to any one of the preceding claims, **characterized in that** it is a foaming detergent composition, such as shampoos, shower gels and bubble baths.

19. Composition according to any one of the preceding claims, **characterized in that** it is a rinse-out or leave-in conditioning composition, a permanent-waving, straightening, dyeing or bleaching composition, or a composition to be applied before or after dyeing, bleaching, permanent-waving or straightening the hair, or alternatively between the two steps of a permanent-waving or hair-straightening operation.

20. Cosmetic process for treating keratin materials, in particular the hair, **characterized in that** it consists in applying to the said materials a composition as defined according to any one of Claims 1 to 19 and then in optionally rinsing with water.

21. Use of a methacrylic acid/C₁-C₄ alkyl acrylate crosslinked copolymer in, or for the manufacture of, a cosmetic composition comprising at least one cyclodextrin and at least one surfactant.

## Patentansprüche

1. Kosmetische Zusammensetzung, **dadurch gekennzeichnet, dass** sie in einem wässerigen Medium mindestens ein Tensid, mindestens ein Cyclodextrin oder eines seiner Derivate und mindestens ein vernetztes Methacrylsäure-C₁-C₄-Alkylacrylat-Copolymer enthält.

2. Zusammensetzung nach Anspruch 1, **dadurch gekennzeichnet, dass** die Cyclodextrine oder eines ihrer Derivate ausgewählt sind unter α-Cyclodextrin, β-Cyclodextrin oder γ-Cyclodextrin oder einem ihrer Derivate.

3. Zusammensetzung nach Anspruch 2, **dadurch gekennzeichnet, dass** die Cyclodextrine unter β-Cyclodextrin oder γ-Cyclodextrin ausgewählt sind.

4. Zusammensetzung nach einem der Ansprüche 1 bis 3, **dadurch gekennzeichnet, dass** das Cyclodextrin oder eines seiner Derivate 0,2 bis 30 Gew.-% und bevorzugt 1 bis 15 Gew.-% ausmacht, bezogen auf das Gesamtgewicht der fertigen Zusammensetzung.

5. Zusammensetzung nach einem der Ansprüche 1 bis 4, **dadurch gekennzeichnet, dass** die Methacrylsäure in dem Copolymer in Mengenanteilen von 20 bis 80 Gew.-% und insbesondere von 25 bis 70 Gew.-% und ganz besonders von 35 bis 60 Gew.-% vorliegt, bezogen auf das Gesamtgewicht des Copolymers.

6. Zusammensetzung nach einem der Ansprüche 1 bis 5, **dadurch gekennzeichnet, dass** das Alkylacrylat in dem Copolymer in Mengenanteilen von 15 bis 80 Gew.-% und insbesondere von 25 bis 75 Gew.-% und ganz besonders von 40 bis 65 Gew.-% vorliegt, bezogen auf das Gesamtgewicht des Copolymers.

7. Zusammensetzung nach einem der Ansprüche 1 bis 6, **dadurch gekennzeichnet, dass** das Alkylacrylat in dem Copolymer unter Methylacrylat, Ethylacrylat oder Butylacrylat ausgewählt ist.

8. Zusammensetzung nach Anspruch 7, **dadurch gekennzeichnet, dass** das Alkylacrylat Ethylacrylat ist.

9. Zusammensetzung nach einem der Ansprüche 1 bis 8, bei der das Methacrylsäure-C₁-C₄-Alkylacrylat-Copolymer mit mindestens einem mehrfach ethylenisch ungesättigten Vernetzungsmittel vernetzt ist.

10. Zusammensetzung nach Anspruch 9, **dadurch gekennzeichnet, dass** der Gehalt an Vernetzungsmittel im Bereich von 0,01 bis 5 Gew.-% und vorzugsweise von 0,03 bis 3 Gew.-% und ganz besonders im Bereich von 0,05 bis 1 Gew.-% variiert, bezogen auf das Gesamtgewicht des Copolymers.

11. Zusammensetzung nach einem der vorhergehenden Ansprüche, **dadurch gekennzeichnet, dass** das vernetzte Methacrylsäure-C₁-C₄-Alkylacrylat-Copolymer in einer Konzentration von 0,01 bis 10 Gew.-%, bezogen auf das Gesamtgewicht der Zusammensetzung, und bevorzugt in einer Konzentration von 0,1 bis 5 Gew.-% vorliegt.

12. Zusammensetzung nach einem der vorhergehenden Ansprüche, **dadurch gekennzeichnet, dass** das Tensid ein anionisches Tensid ist.

13. Zusammensetzung nach einem der Ansprüche 1 bis 12, **dadurch gekennzeichnet, dass** das Tensid in einem Mengenanteil von 0,2 bis 40 Gew.-% und bevorzugt von 1 bis 35 Gew.-%, bezogen auf das Gesamtgewicht der Zusammensetzung, vorliegt.

14. Zusammensetzung nach einem der vorhergehenden Ansprüche, **dadurch gekennzeichnet, dass** sie ferner mindestens ein Konditioniermittel enthält.

15. Zusammensetzung nach dem vorhergehenden Anspruch, **dadurch gekennzeichnet, dass** das Konditioniermittel ausgewählt ist unter Poly-α-Olefinen, fluorierten Ölen, fluorierten Wachsen, fluorierten Gummen, Carbonsäureestern, Siliconen, kationischen Polymeren, Mineralölen, Pflanzenölen oder tierischen Ölen, Ceramiden und Pseudoceramiden sowie Gemischen dieser Stoffe.

16. Zusammensetzung nach dem vorhergehenden Anspruch, **dadurch gekennzeichnet, dass** die Konditioniermittel 0,001 bis 10 Gew.-%, vorzugsweise 0,005 bis 5 Gew.-% und noch bevorzugter 0,01 bis 3 Gew.-% ausmachen, bezogen auf das Gesamtgewicht der Zusammensetzung.

17. Zusammensetzung nach einem der vorhergehenden Ansprüche, **dadurch gekennzeichnet, dass** sie in Form eines Gels, einer Milch, einer Creme, einer Lotion oder eines Schaums vorliegt.

18. Zusammensetzung nach einem der vorhergehenden Ansprüche, **dadurch gekennzeichnet, dass** sie eine schäumende Reinigungszusammensetzung darstellt, wie etwa Shampoos, Duschgele und Schaumbäder.

19. Zusammensetzung nach einem der vorhergehenden Ansprüche, **dadurch gekennzeichnet, dass** es sich handelt um eine nach dem Shampoonieren aufzubringende Pflegespülung, die auszuspülen ist oder nicht, Zusammensetzungen für Dauerwellen, zum Entkräuseln, zum Färben oder zum Entfärben, Zusammensetzungen zur Anwendung vor oder nach einem Färben, einem Entfärben, einer Dauerwellbehandlung oder einer Entkräuselung oder um Zusammensetzungen zur Anwendung zwischen den beiden Schritten einer Dauerwellbehandlung oder einer Entkräuselung.

20. Verfahren zur kosmetischen Behandlung von Keratinmaterialien und insbesondere der Haare, **dadurch gekennzeichnet, dass** es darin besteht, auf diese Materialien eine Zusammensetzung wie in einem der Ansprüche 1 bis 20 definiert aufzubringen und gegebenenfalls anschließend ein Spülen mit Wasser vorzunehmen.

21. Verwendung eines vernetzten Methacrylsäure-C₁-C₄-Alkylacrylat-Copolymers in oder zur Herstellung von einer kosmetischen Zusammensetzung, die mindestens ein Cyclodextrin und mindestens ein Tensid enthält.
